Europäisches Patentamt

European Patent Office

Office européen des brevets

(11) Publication number: 0 488 270 A1

# EUROPEAN PATENT APPLICATION

(21) Application number: 91120362.8

(22) Date of filing: 28.11.91

(51) Int. Cl.⁵: C12Q 1/70, C12N 7/02,
C12Q 1/24, C12Q 1/68

(30) Priority: 30.11.90 JP 341083/90

(43) Date of publication of application:
03.06.92 Bulletin 92/23

(84) Designated Contracting States:
DE GB

(71) Applicant: SHIMADZU CORPORATION
1, Nishinokyo-Kuwabaracho
Nakagyo-ku Kyoto-shi Kyoto 604(JP)

(72) Inventor: Shirasaki, Yosinari
1, Nishinokyo-Kuwabaracho, Nakagyo-ku
Kyoto 604(JP)
Inventor: Ohashi, Tetsuo
1, Nishinokyo-Kuwabaracho, Nakagyo-ku
Kyoto 604(JP)
Inventor: Fukushima, Shigeru
1, Nishinokyo-Kuwabaracho, Nakagyo-ku
Kyoto 604(JP)
Inventor: Tada, Jun
1, Nishinokyo-Kuwabaracho, Nakagyo-ku
Kyoto 604(JP)
Inventor: Yamagata, Koichi
1, Nishinokyo-Kuwabaracho, Nakagyo-ku
Kyoto 604(JP)

(74) Representative: Kahler, Kurt, Dipl.-Ing.
Postfach 1249 Gerberstrasse 3
W-8948 Mindelheim(DE)

(54) Method for collecting the nucleic acid component of virus and method for identifying virus.

(57) A method for collecting the nucleic acid component of virus from a suspension containing virus, comprising the steps of adding a virus adsorptive resin into a suspension thereby capturing the virus on the resin, passing the resin through a filter thereby separating the resin on the filter, and passing a virus solubilizer through the filter to obtain a solution containing the nucleic acid component of virus.

The method may be used in identifying an DNA or RNA virus.

Fig. 2

## FIELD OF THE INVENTION

The present invention relates to a method for collecting the nucleic acid component of virus from a suspension containing the virus and a method for identifying virus employing the method.

## BACKGROUND OF THE INVENTION

(Collection of nucleic acid component of virus)

According to a routine method for collecting the nucleic acid component of virus, a virus-containing sample is solubilized employing an enzyme such as proteinase K, or a surfactant such as sodium dodecyl sulfate, or an alkaline substance such as sodium hydroxide, or a virus-containing cell and virus are crushed with physico-chemical techniques such as sonication, freeze-thawing, or a mill, to firstly obtain a crude product. Then, the extraction with phenol and chloroform and ethanol precipitation are effected to obtain a purified DNA product.

(Method for identifying virus)

Conventional methods for identifying virus include methods for directly detecting etiological microorganisms such as 1. tissue-culture method comprising aseptically infecting culture cells capable of growing a target virus with the virus recovered from a sample and detecting cytopathic effect or plaques; 2. animal-inoculation test method comprising infecting an animal such as a nursing mouse with the virus recovered from a sample and detecting the clinical change of the mouse; 3. hatched egg-inoculation method comprising inoculating the virus recovered from a sample to a hatched egg and detecting lesional effects; or 4. latex agglutination method comprising immobilizing a virus-inherent antibody onto latex beads, adding the immobilized antibody to the sample, and agglutinating the beads via the recognition of the virus by the antibody immobilized on the beads surface; 5. sandwich antibody method comprising bonding a primary antibody to polyethylene plate, affecting this antibody with virus, thereby separating the complex bonded through antigen-antibody reaction, affecting a secondary antibody labeled with an enzyme and a fluorescent substance for B/F separation, and measuring the enzyme activity and fluorescent intensity thereby identifying virus; and 6. DNA probe technique comprising transcribing the nucleic acid component of virus onto a membrane filter, and affecting a specific DNA fragment with a DNA probe labeled with an enzyme and a radioactive substance thereby detecting virus at a genetic level. The present method is a method to detect objective virus and the like by employing the generation of hydrogen bond between a DNA probe and a nucleotide sequence complimentary to the DNA probe, if any, in the DNA of a colony.

A method to indirectly detect etiological microorganisms generally comprises collecting blood from a patient or an infected animal immediately after infection and several weeks after the infection, and examining the increment or decrement of serum antibody levels.

The conventional method described above is not so useful because the culture of microorganisms for more than one week is required for detecting CPE or plaques and because only the viruses of 65 to 75 % of the serum type can grow in general subcultured cells. According to the method comprising inoculating virus to a nursing mouse, viruses of other variable serum types may grow. Because such method requires a longer time and is laborious, it is not commonly employed currently.

Immunological detective methods and serological technique are not satisfactory because plural antigenic functions are observed among serum types.

The approach by hybridization of nucleic acids is extremely promising because viruses of a great number of different serum types can be identified in a far shorter time compared with the time required by tissue-culture methods. However, the approach has not been widely used because the method for extracting viral nucleic acids is complex.

It is a first objective of the present invention to provide a method to obtain the nucleic acid component of virus from a sample suspension in rapid and simple manner, which method can be automated. It is a second objective of the present invention to provide a method for readily identifying virus by employing the aforementioned method.

## SUMMARY OF THE INVENTION

(1) The method for collecting the nucleic acid component of virus according to the first invention is a method for collecting the nucleic acid component of virus from a virus-containing suspension.

The method includes the steps of adding a virus adsorptive resin into a suspension thereby capturing the virus onto the resin, passing the resin through a filter thereby separating the resin on the filter, and passing a virus solubilizer through the filter to obtain a solution containing the nucleic acid component of virus.

According to the present method, the use of a virus adsorptive resin enables the handling of virus as a far larger particle than the virus itself.

Therefore, the handling of virus is easy according to the method. Furthermore, the method can be automated, to obtain the nucleic acid component of virus from a sample suspension in rapid and simple manner.

(2) The method for identifying virus according to the second invention comprises subjecting to polymerase chain reaction (abbreviated as PCR) technique the solution containing the nucleic acid component of virus, obtained by the method of the first invention, and detecting the presence of a desirable DNA virus in the suspension by using the amplified DNA.

A third invention comprises subjecting to the reaction with reverse-phase transcription enzyme the solution containing the nucleic acid component of the RNA virus, obtained by the first method, subjecting the DNA to PCR technique thereby amplifying a DNA complimentary to the RNA of a desirable virus, and detecting the presence of the desirable RNA virus in the suspension.

BRIEF DESCRIPTION OF THE DRAWINGS

Fig. 1 is a flow chart representing a process of the procedure of one embodiment of the present invention;

Fig. 2 (a) depicts the structure of a syringe;

Fig. 2 (b) depicts the structure of a filter unit;

Fig. 2 (c) depicts a view of the syringe with the filter unit mounted thereon;

Fig. 3 is an electrophoreric pattern after carrying out the method of the present invention.

DETAILED DESCRIPTION OF THE INVENTION

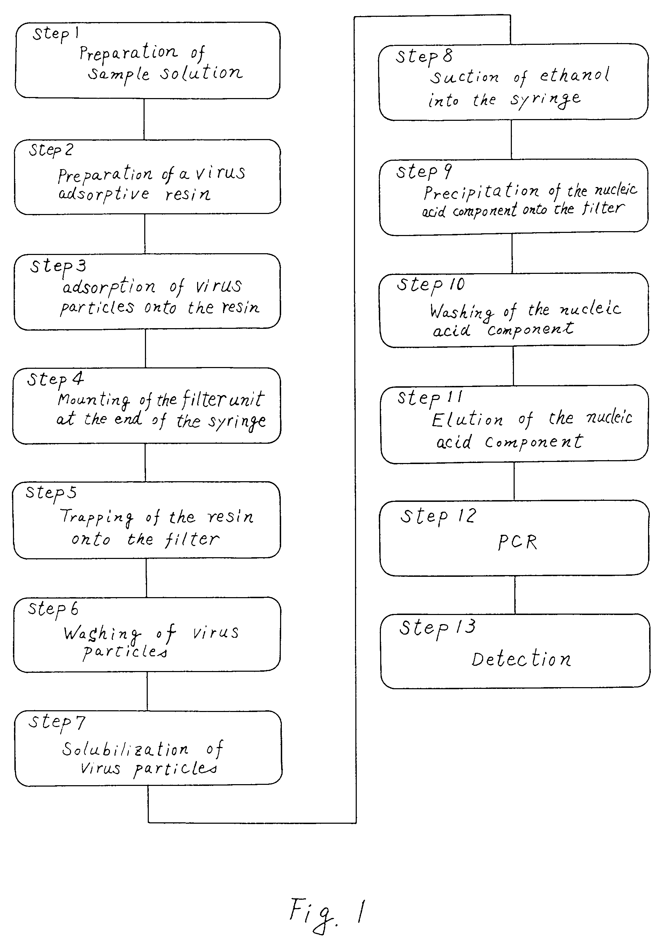

The method for collecting the nucleic acid component of virus according to the present invention will now be explained in the following one embodiment. Fig. 1 represents the process of the procedure of the present invention.

(Preparation of sample solution: Step 1)

In 1 ml of fetal bovine serum was suspended $10^8$ of Sendai virus (murine parainfluenza virus type 1) (prepared by Hayashibara Biochemical Research Institute), to obtain a sample solution.

(Absorptive process: Steps 2 and 3)

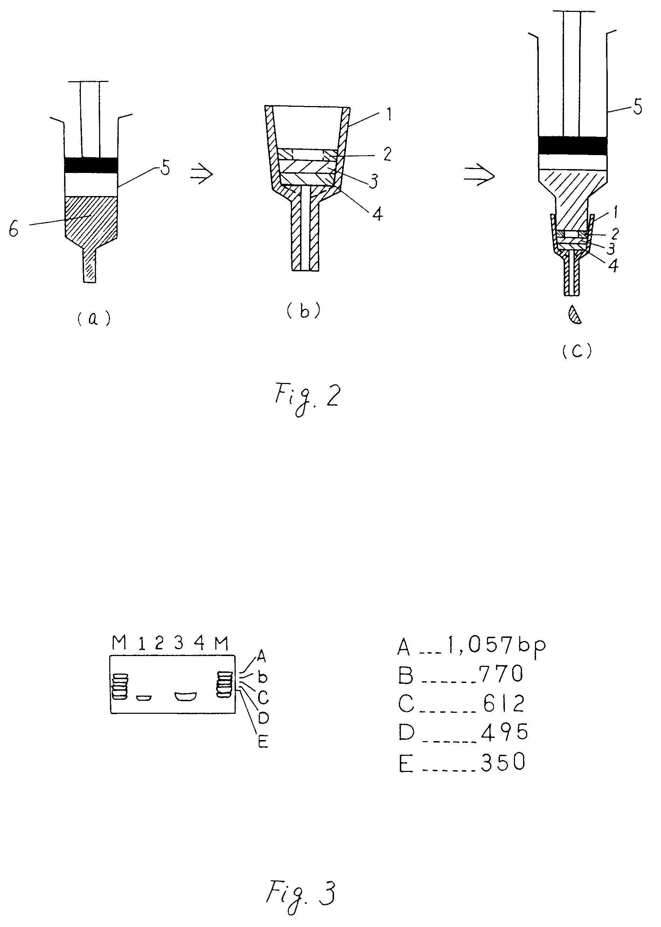

A virus adsorptive resin PB-100 (manufactured by KAO, K.K.) preliminarily washed in water, was added to a sample solution. The resulting mixture was repeatedly subjected to adsorption and discharge process with a 1-ml syringe of Fig. 2(a), to make the virus particles adsorbed onto the resin.

(Washing of virus particle: Steps 4 to 6)

After the adsorptive process, the total volume of the sample solution was suctioned into the syringe, which end was then mounted with a filter unit in Fig. 2 (b), as is shown in Fig. 2(c). Subsequently, the sample solution was discharged to trap the resin onto the filter. In Fig. 2, 1 is the body of the filter unit; 2 is a packing for pressing the filter; 3 is a glass fiber filter GF/B (manufactured by Wattmann, Co. Ltd.); 4 is a glass fiber filter GF/F (manufactured by Wattmann, Co. Ltd.); 5 is a syringe; and 6 is the sample solution. By using the cylinder, 1 ml of purified water was suctioned and discharged twice, for washing off the serum component attached to the virus particles.

(Solubilization of virus particle: Step 7)

A surfactant, aqueous 1% solution of SDS (200 $\mu$l), was suctioned into the syringe described above, and was passed through the inside of the filter unit.

(Precipitation of the nucleic acid component: Steps 8 to 10)

A mixed solution (420 $\mu$l) of 400 $\mu$l of ethanol and 20 $\mu$l of aqueous 3 M sodium acetate solution was suctioned into the syringe at 0 °C. The solution was discharged from the syringe, to immobilize the nucleic acid component in precipitated state onto the filter. By means of the suction and discharge process with 1 ml of 70 % ethanol at 0 °C and the suction and discharge with 1 ml of 99 % ethanol, the precipitate of the nucleic acid component was washed.

(Elution of nucleic acid component: Step 11)

One hundred $\mu$l of an aqueous solution of 0.5 % Tween 20 and 0.5 % Nonidet P-40 was suctioned into the syringe and passed through the filter unit, followed by the incubation at 60 °C for 10 minutes. Then, the solution was discharged to obtain a solution of the nucleic acid component.

(Reverse-phase transcription and amplification of complimentary DNA: Step 12)

Three $\mu$l of the eluate was separated, which was then subjected to the reverse-phase transcription reaction for synthesizing a complimentary DNA to a specific RNA region and simultaneously subjected to PCR technique as one method for specific amplification of an objective DNA (Saiki, R.K., et al., Science, 239, 487-491 (1988)).

Fifty $\mu$l of a mineral oil as a preventive agent against vaporization was layered on 30 $\mu$l in total of 12.2 $\mu$l of distilled water, 2.5 $\mu$l of each of two primers (20 $\mu$M), 4.8 $\mu$l of dNTP (1.25 mM of each dNTP), 0.5 $\mu$1 of a reverse transcriptase (200 units/ml; manufactured by BRL), 1 $\mu$l (40 units/ml) of RNasin (manufactured by TOYOBO, K.K.), 0.5 % Nonidet P-40, 3 $\mu$l of an aqueous 0.5 % solution of Tweetn 20, 3 $\mu$l of 10-fold buffer (manufactured by Perkin-Elmer-Cetus, Co. Ltd.), and 1.5 unites of Taq polymerase (manufactured by Perkin-Elmer-Cetus, Co. Ltd.). The resulting layered mixture was subjected to a DNA Thermal Cycler (manufactured by Perkin-Elmer-Cetus, Co. Ltd.). The term "primer" herein referred to is a 20 bp oligonucleotide. The term "10-fold buffer" herein is 100 mM Tris-HCl buffer (pH 9.0), 500 mM KCl and 15 mM $MgCl_2$.

The conditions for reverse-phase transcription were at 42 °C for 40 minutes, while the temperature cycle of PCR was at 94 °C for 1 minute for modification, at 50 °C for 1 minute for annealing and at 72 °C for 1 minute for chain polymerization. Forty-two cycles of each 5.7-minute cycle were carried out.

(Detection: Step 13)

Ten $\mu$l of the solution after the PCR procedure described above was separated, and subjected to electrophoresis at 100 V for 35 minutes on 2 % agarose gel containing ethidium bromide. The gel placed on a transilluminator was subsequently irradiated with UV ray of a wave length of 320 nm. Fluorescence emitted from the PCR-amplified DNA. The fluorescence was imaged on an instant film mounted on a camera. The results are shown in Fig. 3. The method in accordance with the present invention was applied to the suspension of a sample containing Sendai virus. The electrophoretic pattern thereof is depicted in lane 1 of Fig. 3. Consequently, a band appeared at an anticipated position of 173 bp. This position was identical to the position of a positive control sample (lane 3 of Fig. 3), simultaneously electrophoresed, which suggests that Sendai virus can be detected specifically. The term "positive control" means a sample employing a DNA solution, obtained by solubilizing Sendai virus with a surfactant, followed by the extraction with phenol and chloroform and ethanol precipitation.

Furthermore, lane 2 shows a pattern obtained by processing serum to which virus intentionally was not added, in the same manner as the case of a sample solution; lane 4 shows a pattern obtained by PCR technique without adding eluate; M is a molecular-weight marker, which is obtained by complete digestion of $\phi$X174DNA with a restriction enzyme HincII; and A, B, C, D and E are the corresponding numbers of base pairs, individually.

The results described above indicate that serum Sendai virus can be detected at a short time of about 5 hours in simple manner, according to the method of collecting the nucleic acid component of virus and the method of identifying virus, in accordance with the present invention.

**Claims**

1. A method for collecting the nucleic acid component of virus from a suspension containing virus, comprising the steps of adding a virus adsorptive resin into a suspension thereby capturing the virus on the resin, passing the resin through a filter thereby separating the resin on the filter, and passing a virus solubilizer through the filter to obtain a solution containing the nucleic acid component of virus.

2. A method for identifying DNA virus comprising the steps of subjecting to polymerase chain reaction (abbreviated as PCR) technique the solution containing the nucleic acid component of virus obtained by the method claimed in claim 1, thereby amplifying a desirable DNA of the virus and detecting the presence of the desirable DNA virus in the suspension by using the amplified DNA.

3. A method for identifying RNA virus comprising the steps of subjecting to the reaction with a reverse transcriptase the solution containing the nucleic acid component of the RNA virus obtained by the method claimed in claim 1, subjecting the DNA to PCR technique thereby amplifying a DNA complimentary to the RNA of a desirable virus, and detecting the presence of the desirable RNA virus in the suspension.

Step 1
Preparation of
Sample solution

Step 2
Preparation of a virus
adsorptive resin

Step 3
adsorption of virus
particles onto the resin

Step 4
Mounting of the filter unit
at the end of the syringe

Step 5
Trapping of the resin
onto the filter

Step 6
Washing of virus
particles

Step 7
Solubilization of
virus particles

Step 8
Suction of ethanol
into the syringe

Step 9
Precipitation of the nucleic
acid component onto the filter

Step 10
Washing of the nucleic
acid component

Step 11
Elution of the nucleic
acid component

Step 12
PCR

Step 13
Detection

Fig. 1

Fig. 2

A --- 1,057 bp
B ------ 770
C ------ 612
D ------ 495
E ------ 350

Fig. 3

European Patent Office

**EUROPEAN SEARCH REPORT**

Application Number

EP    91 12 0362

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | EP-A-0 366 448 (GENERAL HOSPITAL CORPORATION)<br>* abstract *<br>* page 5, line 27 - line 37 *<br>--- | 1-3 | C12Q1/70<br>C12N7/02<br>C12Q1/24<br>C12Q1/68 |
| Y | GB-A-2 024 421 (BOEHRINGER MANNHEIM GMBH)<br>* the whole document *<br>especially<br>* claims 24,25 *<br>--- | 1-3 | |
| A | PATENT ABSTRACTS OF JAPAN<br>vol. 13, no. 388 (C-630)(3736) 28 August 1989<br>& JP-A-1 137 999 ( SHIMADZU CORP ) 30 May 1989<br>* abstract *<br>--- | 1-3 | |
| A | EP-A-0 145 356 (NATIONAL RESEARCH DEVELOPMENT CORPORATION)<br>--- | | |
| P,X | JOURNAL OF VIROLOGICAL METHODS<br>vol. 31, February 1991, AMSTERDAM NL<br>pages 139 - 146;<br>I. KORSCHINECK ET AL.: 'A PCR membrane spot<br>assay for the detection of plum pox virus RNA in<br>bark of infected trees'<br>* abstract *<br>----- | 1-3 | **TECHNICAL FIELDS SEARCHED (Int. Cl.5)**<br><br>C12Q |

The present search report has been drawn up for all claims

| Place of search | Date of completion of the search | Examiner |
|---|---|---|
| THE HAGUE | 28 FEBRUARY 1992 | MOLINA GALAN E. |